# EUROPEAN PATENT APPLICATION

(11) **EP 1 900 319 A1**
(43) Date of publication of application: **19.03.2008**
(21) Application number: 06716850.0
(22) Date of filing: 27.01.2006
(51) Int. Cl.: A61B 3/113, A61B 5/0496

(54) **METHOD FOR TESTING A SPATIAL APTITUDE ANOMALY AND DEVICE FOR CARRYING OUT SAID METHOD**

(30) Priority: 09.06.2005 US 147706
(71) Applicant: Pugach, Vladimir Nikolaevich, Izhevsk, 426076 (RU); Tikhonov, Vitaly Viktorovich, Mountain View, CA 94040 (US); Tikhonova, Svetlana Viktorovna, Mountain View, CA 94040 (US)
(72) Inventor: Pugach, Vladimir Nikolaevich, Izhevsk, 426076 (RU); Tikhonov, Vitaly Viktorovich, Mountain View, CA 94040 (US); Tikhonova, Svetlana Viktorovna, Mountain View, CA 94040 (US)
(74) Representative: Jordan, Volker Otto Wilhelm
(86) International application number: PCT/RU2006/000025
(87) International publication number: WO 2006/132566

(57) **Abstract**

There have been developed a method for detecting spatial perception anomalies and a device for carrying out this method. The device comprises an arrangement (1) for fixing the head of a person under test, a first transparent screen (2) disposed in a close-vision field, and a second screen (3) seen through the first one and located in a far-vision field of the person under test. The device is provided with means (5) and (6) for creating images selectively on the first and second screens, and also with a means (4) for recording trajectories of the movement of the eye pupil of the person under test while following these images. The method is based on detecting specific irregularities in trajectories of the eye pupils, such as nystagmuses, cut-offs, sudden drops of the gaze, etc., when the person under test follows the smooth trajectory of a moving image on one of the screens; and also on finding irregular changes in the size and position of the eye pupil in response to instantaneous switching of images between the screens while the head of the person under test is kept stationary. 2 independent claims, 14 dependent claims, 9 illustrations.

## Description

### Field of the Invention

The present invention relates to the field of neuropsychology, psychophysiology and neurophysics of the brain, and can be used for detecting anomalies in spatial perception that may be caused by brain disorder and damage, stress, tiredness and psychological traumas.

### Background of the Invention

It is known that some inborn brain defects, disorders, or damage may cause anomalies of spatial perception and attention disorders that in some professions may lead to increase in errors to the extent that the individual becomes unsuitable for the profession. Examples of such professions that may require extraordinary attention are pilots, air-traffic controllers, vehicle drivers, etc.

The problems associated with anomalies in spatial perception concern not only people involved in professions where lack of attention may lead to high-risk situations and even to fatal consequences, but also to events of the everyday life such as studying, operation on machine tools, computer programming, management, etc. In view of the above, it is important in some fields of human activities to test the individuals for fitness to their professions or types of work they conduct.

The method and device of the invention serve as a tool for testing children, e.g., with the attention deficit hyperactivity disorder syndrome (ADHD). Many methods and devices are known in the art for testing spatial perception skills and thus for detecting fitness to the profession or activity.

For example, U.S. Pat. No. 4,973,149 discloses a system for eye movement detection that utilizes an IR light emitting diode mounted coaxially in front of the lens of an infrared sensitive video camera. This system allows remotely making images of the eye of a computer operator. The reflected light causes bright eye effect which outlines the pupil as brighter than the rest of the eye and also causes an even brighter small glint from the surface of the cornea. The computer includes graphic processing which takes a video image, digitizes it into a matrix of pixels and analyzes the matrix. Using special algorithms, the analysis determines the location of the pupil's center and the location of the glint relative to each other and with this information determines where the eye is gazing. If the eye-gaze is for a predetermined time at images in selected areas on the computer screen, the area is selected and results in actuation of other devices or the presentation of an additional image on the screen.

U.S. Pat. No. 5,555,895 describes a process and a device for analyzing the movement of the eye or eyes of a patient, i.e., of a human being or animal. A device comprises two video cameras mounted on a spectacle frame and rigidly connected together and form part of a removable module; said first and second cameras have points of sight of their respective scenes which are close.

U.S. Pat. No. 5,422,690 discloses a fitness impairment tester that implements a self-administered screening test to determine whether a subject is physically impaired. The tester is fully automated to respond to the entering of a subject's personal identification on a keypad. By superposing two different colored lights as viewed through an eyepiece, the subject aligns his or her eye pupil on the optical axis of pupil imaging optics which focuses an image of the subject's pupil on an image plane. The tester automatically recognizes this condition and audibly signals the subject that the test is about to begin. Light stimuli are provided to cause the pupil to change size and the eye to move. Pupil diameter measurements are made of the image of the subject's pupil in response to on axis light stimuli. Eye tracking of the subject's eye in response to moving light stimuli provide a measure of eye movements. The data acquired by measuring pupil diameter and saccadic movements of the subject's eye as a function of time are compared with baseline data for the subject stored in a database, and an output indicative of a deviation of said current measurements from said baseline data is provided.

U.S. Pat. No. 5,668,622 describes a device for measuring the position of the fixing point (P') of an eye on a video screen. The device comprises: a mobile support positioned close to the eye and on which first illumination means and a video camera are fixed so as to embody images of the eye, and a fixed support distant from the eye and on which the video screen orientated opposite the eye and a plurality of luminous sources illuminating the eye in alternative with the first illumination means are positioned and disposed around the video screen so as to create on the eye corneal reflections and means for treating the images obtained from the eye. The description also discloses an eye illumination method in which the eye is illuminated by flashes each emitted at the end of each taking of images, as well as an application of the device for displaying on the video screen images which change according to the movements of the eye wherein a high resolution zone (Z) is defined around the fixing point (P') and for moving this zone according to the movements of the eye.

However, none of the inventions mentioned above provide testing of an individual with regard to anomalies in spatial perception thereof.. Furthermore, none of the devices mentioned above allow complete analysis of static and dynamic phenomena that occur in the spatial perception in switching the gaze on an object from the close-view field to the far-view field, or vice versa.

### Essence of the Invention

It is an obj ect of the invention to provide a universal method and device for testing individuals with regard to their spatial perception anomalies which cannot be detected by other known methods and devices. One more object of the invention is to provide a method and device for determining the fitness of different individuals to a specific profession, especially for professions associated with high risks. A further object is to provide testing of the individuals with regard to spatial perception on the basis of personalized data bases accumulated for specific activities and for specified individuals.

Said objects are accomplished owing to the fact that a method for testing spatial aptitude anomalies comprises: fixing the head of an individual under test, arranging two screens in front of the person under test, the first of which screens is made transparent and the second of which screens is disposed behind the first screen in a direction of the person under test's gaze; creating moving images on the screens; registering the movement of at least one eye pupil of the person under test; comparing the trajectory of the image movement on the screens with the trajectory of the movement of at least one eye pupil of the person under test and, depending on the deviation of the compared trajectories, a conclusion being drawn about the presence of anomalies in the spatial aptitude of the person under test.

Movable images on the screens are created separately on the first screen and on the second screen with the image reciprocating between two points along a rectilinear trajectory or with the image reciprocating between two points along a curvilinear trajectory, or with the image moving along a closed trajectory.

Movable images on the screens can be created with instantaneous switching of the images between the first and second screens, wherein the movable images on the screens may be movable light spots.

The first screen is removed from the eyes of the person under test to a distance of from 1 cm to 60 cm.

Furthermore, said objects are accomplished with the aid of a device for carrying the inventive method into effect, which device comprises: an arrangement for fixing the head of a person under test; a first transparent screen located at first distance from the arrangement for fixing the head of the person under test; a second opaque screen located at a second distance from the arrangement for fixing the head of the person under test, the second distance being larger than the first distance; a means for creating an image on the first screen; a means for creating an image on the second screen; and a means for registering the movement of at least one of the person under test's eye pupils.

The means for creating the first image and the means for creating the second image are embodied with the possibility of creating movable images.

The first screen may be made flat and the second screen may have a curved shape with its concave side facing the arrangement for fixing the head of the person under test, the first screen being disposed at a distance of from 1 cm to 60 cm from the arrangement for fixing the head of the person under test.

Furthermore, the device can be provided with a processor associated with the means for creating an image on the first screen, with the means for creating an image on the second screen and with the means for registering the movement of at least one of the person under test's eye pupils.

### Brief Description of the Drawings

FIG. 1 is a schematic side view of the device of the invention;
FIG. 2 is a view illustrating a trajectory of a light spot on the screen and an associated trajectory of the eye pupil;
FIG. 3 shows a nystagmus (involuntary movement of the eye) that may occur as an anomaly of spatial perception in the test shown in FIG. 2;
FIG. 4 shows an anomaly of spatial perception that is revealed as a chordal cut-off in the oval trajectory of the pupil movement.
FIG. 5 illustrates a test with linear movements of the light spot in direct and reverse directions;
FIG. 6 illustrates an anomaly revealed in sudden jump of the gaze in the downward or upward direction;
FIG. 7 shows a curvilinear trajectory of the eye pupil in following linear movements of the light spot;
FIG. 8 shows a nystagmus that may occur in the test of FIG. 6;
FIG. 9 is a schematic view of a test performed by rapid switching of the image between two screens.

### Disclosure of the Invention

The invention is based on the common knowledge in the field of human physiology and on the inventors' observations that in the visual perception each individual uses the close-vision field and the far-vision field that exist irrespective of our consciousness. For example, when an individual is reading a book or looking at a computer keyboard, he/she uses the close-vision field. The inventors have found that in an adult person on average the close-vision field is located at a distance of up to 30 - 60 cm from the observer's eye, and the center of the close-vision field is generally below the horizontal plane passing through the person under test's eye pupil, approximately 15° below the aforementioned horizontal plane. The far-vision field is extended from the close-vision field to infinity, e.g., from 40 - 60 cm to infinity. Examples of the far-vision field are situations on the road during driving, a blackboard in a class-room, a football field, the control panel of an air-traffic controller, the control panel of an operator of a nuclear power station, etc. A person under test cannot simultaneously see objects distinctly in both fields, and in switching from one field to another the person under test changes the so-called plane of accommodation and angle of observation.

The processes of visual perception of objects located in the close-vision field (hereinafter referred to as "close field") and in the far-vision field (hereinafter "far field") are different and have a complicated psychophysiological nature. With regard to the psychophysiological processes that occur in viewing objects, all individuals can be roughly divided into the following four categories:
1) people who have normal spatial perception in the close field and far field;
2) people who have normal spatial perception in the close field but abnormal perception in the far field;
3) people who have abnormal spatial perception in the close field but normal perception in the far field; and 4) people who have abnormal spatial perception in the close field and abnormal perception in the far field.

The aforementioned anomalies can be revealed in specific movements of the person under test's eye pupils, e.g., in the horizontal, vertical, or orbital movements of the eye pupils. This is true for observing the objects in both the close field and the far field. In the medicine such movements of the eye pupils are known as "saccadic nystagmatic movements", and in the neurophysics such movements of the eye pupils are known as "saccadic nystagmus".

Saccades or jump-like movements are used to bring the eye rapidly from one point of regard to another. As one does not see during a saccade, it is best to get them over as quickly as possible. Accordingly, saccades typically move at speeds between 200 and 600 degrees/sec. To move gaze 90 degrees, it takes 1/3 second (which still seems like a rather long time not to see).Things that happen very quickly often have a tendency to get out of control. With saccades, most disorders consist of either instability (oscillation, flutter and opsoclonus), or inability to inhibit saccades (square wave jerks, saccadic intrusions). Methods of recording saccadic nistagmuses are known and described, e.g., by Timothy C. Hain in the article "Saccadic Nystagmus" (see http://www.dizziness-and-balance.com/practice/saccadic-nystagmus.htm).

One more specific anomaly has been found, that may occur in spatial perception in some individuals. This anomaly may be called a "phenomenon of a broken mirror". This phenomenon is revealed as a discontinuity of vision in the peripheral areas of the aforementioned fields, especially in rapid switching from one field of vision to another. The present invention is based on the fact that the pattern of the eye pupil movements is related to disorders in the spatial perception. Another found anomaly in the close-vision or far-vision field is the so-called "sudden slide of the gaze in the upward or downward direction".

A patient to be tested is sat in a chair and his/her head is fixed against movements so that only the eyes can follow the object. The object may comprise a moving light signal of any configuration and sufficient intensity of brightness. The light signal is first projected onto a close screen located in the area of the aforementioned close field. When the person under test's eye pupils follow the moving light signals, movements of the eye pupils are registered, e.g., by means of an image sensor. Such registrations are carried out separately for the observations in the close and far fields. If necessary, without changing positions of the head of the person under test and without interrupting the eye movement registration, projection of the light signal is switched to the far screen located in the aforementioned far field. Movements of each eye and changes in the size of the pupils are registered individually and the results of the registration can be recorded in a computer and later compared for both eyes, as well as with the previously accumulated data for detecting the dynamics of changes in the person under test's spatial perception. Light signal trajectories may be different and may be distinguished for diagnosing different spatial perception disorders or for other purposes. More specifically, the patterns of the normal perception are compared with the patterns of abnormal perception and the comparison will distinctly reveal the presence or development of the disorder.

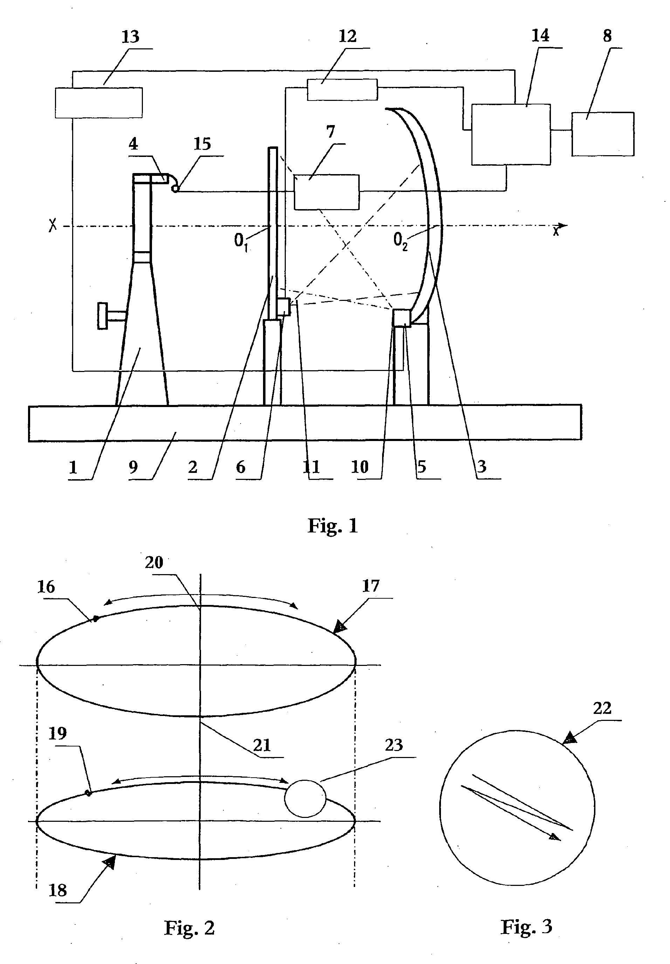

The described method is realized with the use of a device shown in Figure 1, that comprises: an arrangement 1 for fixing the head of the person under test; a transparent first screen 2 located in the close field at a small distance from the arrangement 1 so that a horizontal axis should pass precisely through the person under test's eye and through the center of the first screen 2; a second screen 3 that is opaque, located over a distance from the arrangement 1 in the far field and being aligned with the first screen 2. The device also comprises a means 4 for registering positions of the person under test's eye pupils; a means 5 for creating an image on the first screen; a means 6 for creating an image on the second screen 3; a data acquisition unit 7; a data indication or display unit 8. The first screen 2 is made flat and the second screen 3 has a curved shape with its concave side facing the arrangement 1 for fixing the head of the person under test.

The arrangement 1 for fixing the head of the person under test, the transparent first screen 2 and the second screen 3 are secured to a base support 9 so that the horizontal axis X-X should pass through the point in the middle between the eyes of the person under test (not shown in the drawing) whose head is fixed in the arrangement 1, and also through the geometrical centers O₁ and O₂ of the first and second screens, respectively.

The means 5 for creating an image on the first screen is embodied as a laser beam projector 10 intended for projecting moving images onto the first screen 2. The projector 10 may be located in any position convenient for projecting moving images, in particular, light spots, onto the first screen 2. As is shown in Fig. 1, the projector 10 is located in a position between the first screen 2 and the second screen 3. A second laser projector 11 is intended for projecting an image onto the second screen 3. This projector also may be positioned in any location convenient for obtaining a relatively undistorted moving image, i.e. to project a light spot onto the screen 3. As is shown in FIG. 1, the projector 11 is positioned near the edge of the first screen 2. Furthermore, the projectors 10 and 11 are located in positions that exclude direct exposure of person under test's eyes to the laser beams. Moreover, the intensity of the light reflected and scattered from the images created on the screens should be within the limits specified by appropriate standards and safety codes, e.g., standards of NBS USA.

The image generated by the projectors 10 and 11 is controlled by controllers 12 and 13, respectively, for controlling their operation under commands of a central processing unit 14 that is connected to both controllers.

The device is equipped with a means (image sensors) for continuous tracking movements and recording images of the eye pupils. Since the sensors for tracking movements of the eye pupils are identical for both eyes, only one of them, i.e., the image sensor 15 is shown. The image sensor 15 should be positioned in a location that provides unobstructed view of the person under test's eyes. The image sensor 15 is connected to the central processing unit 14 via the data acquisition unit 7 that receives the data from the image sensor 15 and transmits them to the central processing unit for further processing.

The arrangement 1 for fixing the head of the person under test is a device conventionally used in any ophthalmologic equipment, e.g., of the type described in U.S. Pat. No. 5,689,325. As usual, the head support is provided with mechanisms for adjusting the position of the head support, and, hence of the person under test's eyes

The eye pupil position data obtained by the sensor 15 are used to register eye motion during the test. Such sensors are known (see, e.g., U.S. Pat. No. 6,334,683) and are usually based on the use of a video camera with a tracking system which automatically recognizes and tracks the position of the eye, being geared to landmarks of peculiar kind present within an image of a human eye. Such a system requires illumination of the eye, e.g., by infrared rays. Infrared (IR) radiation typically with 850 to 930 nm wavelength is used because it provides a good picture contrast between the pupil and iris. Additionally, the use of IR radiation decouples the lighting source from other optical sources which do not contain the infrared wavelengths.

An image of the eye, illuminated by invisible infrared radiation from an IR light source, is constructed by the tracking sensor 15 with the help of an IR-sensitive video camera. Under normal conditions, the pupil of the eye appears as a dark hole to the illumination. The dark pupil image data come to the central processing unit 14 via the data acquisition unit 7. The central processing unit 14 is connected to a display unit 8 that can visually display the results of the test.

A description of the eye illumination device and position thereof with respect to the person under test's eye on the head fixing support can be found, e.g., in U.S. Pat. No. 5,668,622.

The first screen 2 can be made from a transparent material of high light transmissivity, e.g., no less than 99.0%. This screen may be flat or slightly negatively curved (e.g., with the radius of 30 to 60 cm) with respect to the moving image generating projector 10 and can be made from glass or plastic and may have a coating capable of partially absorbing and scattering the light of a predetermined wavelength, e.g., 632.8 nm, or 532 nm, i.e., of the light emitted by the movable image generating laser. The screen should provide clear view of the images on the far screen from the position of the person under test's eyes when his/her head is fixed in the arrangement 1.

The second screen 3 may be transparent or opaque and can be made from glass, plastic, or other materials. For example, it can be made from an opaque glass. The opaque material is preferable. The screen 3 may be colored in order to enhance the color contrast and ensure better perception of the moving image. The screen 3 may have a coating that ensures partial absorption and scattering of the incident light. The far screen has a curvilinear profile shown in FIG. 1. It may be spherical, paraboloid of revolution, or may have any other suitable profile. The average radius of curvature may be of about 80 cm to 150 cm. These numbers should not be construed as limiting the scope of the invention and are given only as an example.

The moving image generating projectors 10 and 11 comprise laser light sources and light beam deflecting systems that may emit light beams that in a static state produce on the respective screens light spots having a diameter of 2 to 5 mm. Movements of the light beams are controlled by means of so-called electro-optical image generator or laser image generators with moving heads that control positioning of the light spot within a wide range of movement speeds and with possibility of coordinating positions of the light beam spots. Such laser light sources are commercially produced (see, e.g., International Laser Production, http://www.internatlaser.com/DPSS;LASER SYSTEMS.html). The above-mentioned light sources may produce light spots moving from minimal speeds to high speeds undetectable by a human eye. For the purposes of the invention, the optimal speed for moving the light spot on the first screen 2 and on the second screen 3 should be within the range of 1 cm/sec to 100 cm/sec.

The test of spatial perception shown in FIG. 2 can be carried out by projecting a moving object (a light spot) onto the first screen 2 or second screen 3 (FIG. 1) with the help of the projectors 10 or 11. The upper part of FIG. 2 shows an example of movement of a light spot 16 that moves along a selected trajectory - along an oval shown with an arrow. The lower part of FIG. 2 shows the trajectory 18 of the of the person under test's eye pupils registered when the person under test's eyes follow the travel of the light spot 16. In the case of normal perception, the trajectory should be similar to the trajectory 17, i.e., free of nystagmatic movements.

The test is started from a predetermined position of the light spot -- from point 20. The light spot 16 first moves in a clockwise direction with a predetermined speed, from point 20 to point 21. The movement of the light spot 16 is then discontinued for a time sufficient for the eye to accommodate for the next observation (about 0.8 to 1.2 sec.). If necessary, the person under test may be asked if he/she is ready for the next step of the test. The movement of the light spot 16 is then resumed from the same point 20 to the point 21, but in the counterclockwise direction. In both movements, the pattern of the eye pupil movements are tracked and recorded by means of the sensor 15 (FIG. 1). The recorded data are transmitted to the central processing unit 14 and, if necessary, displayed on the display unit 8.

In the case of a normal spatial perception, the trajectory 17 of the eye pupil movements will be free of any nystagmuses on any part of the trajectory 17. FIG. 3 shows on a larger scale a nystagmus 22 that occurred in the zone 23 of FIG. 2. Although this nystagmus may comprise reciprocal movements of the eye pupil along the same line, for simplicity of explanation the nystagmatic movements are shown as a zigzag line. Such nystagmus means that at a certain period of time the person under test does not see the light spot 16 and therefore may be evaluated as a person having a spatial perception deficit.

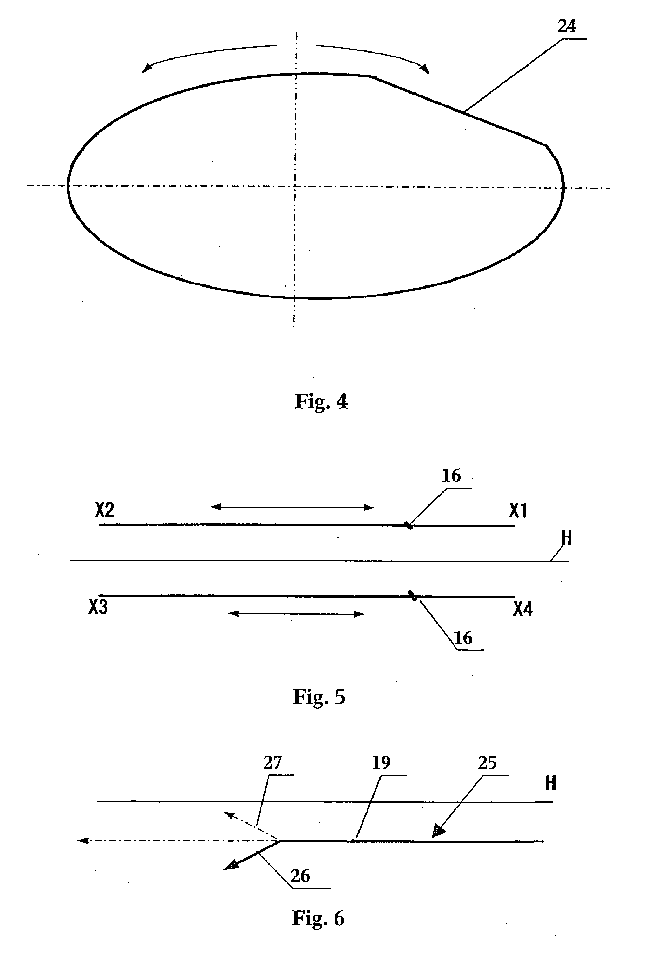

Another example of the same test as in FIG. 2 may indicate another defect of the spatial perception that is shown in FIG. 4. In the case of spatial perception deficit the person under test may develop a cut-off in following a smooth trajectory of the type as 17 (FIG. 2). This "cut-off" (straightening of the trajectory) is shown in FIG. 4 as a chordal line 24. The cut-off may occur on any part of the trajectory of the eye pupil.

Figures 5 - 8 illustrate the test which is similar to the one shown in FIG. 2 but with linear reciprocal movements of the light spot. Line H designates a horizontal plane that contains line X-X shown in FIG. 1, i.e., the line that passes through the middle point between the eyes of the person under test and the centers of the screens 2 and 3. In that case, in FIG. 5, the line X1-X2 will depict the trajectory of the light spot 16 on the second screen 3, and the line X3-X4 will depict the trajectory of the light spot 16 on the first screen 2. It is easy to note that the horizontal plane designated by line H is located between said trajectories.

In this test, the light spot 16 starts its movements point X1 in the forward direction (from right to the left in FIG. 5) and then in a reverse direction from point X2 (from left to the right in FIG. 5).

In FIG. 6, line 25 designates the trajectory of movement of the eye pupil 19 in following the movement of the light spot 16 on the first screen 2 (FIG. 1). A defect that can be revealed in this test is usually manifest as a sudden deviation of the gaze in the downward direction (trajectory 26) or in the upward direction (trajectory 27). This phenomenon occurs predominantly in testing gaze movements only on the close-field screen. The normal spatial perception will correspond to a straight-line trajectory on both screens without symptoms of sudden deviation.

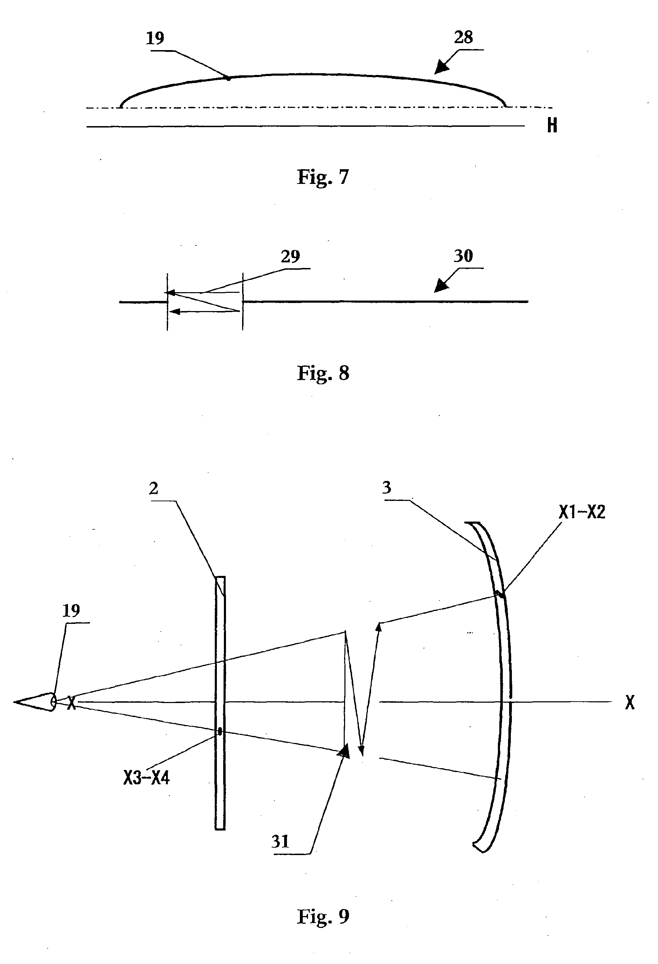

In FIG. 7, the curved trajectory 28 shows another anomaly that, in response to the linear movement of the light spot 16 (FIG. 5) in the forward and reverse direction, is reveled as curvilinear movement of the eye pupil 19.

FIG. 8 illustrates another anomaly in the spatial perception, which is similar to the one shown in FIG. 3 but consists in a linear nystagmoidal movement shown in the form of zigzag line 29. In fact, the zigzag line is shown conventionally, as the movement may comprise reciprocations along the same straight line. There may be more than one nystagmus on the same rectilinear trajectory 30 shown in FIG. 8.

FIG. 9 shows the spatial perception test that allows revealing anomalies in switching the gaze from the first screen 2 of the close-vision field to the second screen 3 and vice versa. This test is fulfilled by instantaneously switching the image from one screen to the other. This is applicable to any tests described above with reference to FIGS. 2 to 5, but without changing the position of the head of the person under test. The light spot may be movable or stationary but the switching should instantaneous. The person under test's response is measured by tracking the trajectory and recording the time and the movements of the eye pupil 19 in following the light spot. If necessary, the response may be controlled by recording and measuring changes in the positions and diameter of the eye pupil. Methods of measuring the diameter of the eye pupil are known in the art and are mentioned, e.g., in U.S. Pat. No. 5,422,690. In FIG. 9, point X1-X2 corresponds to line X1-X2 in FIG. 5, and point X3-X4 corresponds to line X3-X4 in FIG. 5. Said anomaly is revealed as nystagmus 34 during which the object is not seen by the person under test.

### Industrial Applicability

Thus, the inventive method and device allow revealing anomalies in spatial perception, which are undetectable using other methods and instruments and which can be employed for determining professional fitness of a particular individual to a specific profession, especially for professions associated with high risks. The invention also can be used for testing the individuals with regard to spatial perception on the basis of personalized data bases accumulated for specific activities and for particular individuals.

## Claims

1. A method for detecting anomalies in spatial perception, **characterized in that** the head of a person under test is fixed in a stationary state, two screens are disposed in front of said person, a first of which screens is embodied transparent and a second screen is located behind the first screen in a direction of said person's gaze; movable images are created on said screens; movements of at least one eye pupil of said person are recorded; the trajectory of the travel of the images on the screens is compared with the trajectory of the movement of at least one eye pupil of said person and depending on the deviation of the trajectories being compared a conclusion is drawn about the presence of anomalies in spatial perception of said person.

2. The method of claim 1, **characterized in that** movable images on the screens are created separately on the first screen and on the second screen with the image reciprocating between two points along a rectilinear trajectory or with the image reciprocating between two points along a curvilinear trajectory, or with the image moving along a closed trajectory.

3. The method of anyone of claims 1 or 2, **characterized in that** movable images on the screens are created with instantaneous switching of said images between the first and second screens.

4. The method of anyone of claims 1 or 2, **characterized in that** the first screen is removed from the eyes of the person under test to a distance of from 1 cm to 60 cm.

5. The method of claim 3, **characterized in that** the first screen is removed from the eyes of the person under test to a distance of from 1 cm to 60 cm.

6. The method of anyone of claims 1 or 2, or 5, **characterized in that** the movable images on the screens are movable light spots.

7. The method of claim 3, **characterized in that** the movable images on the screens are movable light spots.

8. The method of claim 4, **characterized in that** the movable images on the screens are movable light spots.

9. A device for carrying out the method of claim 1, **characterized in that** it comprises an arrangement for fixing the head of a person under test in a stationary position; a first screen which is embodied transparent and is located at a first distance from the arrangement for fixing the head of said person under test; a second screen which is embodied opaque and is located at a second distance from the arrangement for fixing the head of said person under test, wherein the second distance is greater than the first distance; a means for creating an image on the first screen; a means for creating an image on the second screen; and a means for recording movements of at least one eye pupil of said person under test.

10. The device of claim 9, **characterized in that** the means for creating the first image and the means for creating the second image are embodied with the possibility of creating movable images.

11. The device of anyone of claims 9 or 10, **characterized in that** the first screen has a flat shape, and the second screen has a curvilinear shape with a concave side facing the arrangement for fixing the head of the person under test.

12. The device of claim 11, **characterized in that** the first screen has a flat shape, and the second screen has a curvilinear shape with a concave side facing the arrangement for fixing the head of the person under test.

13. The device of anyone of claims 9 or 10, or 13, **characterized in that** the first screen is located at a distance of 1 cm to 60 cm from the arrangement for fixing the head of a person under test in a stationary position.

14. The device of claim 11, **characterized in that** the first screen is located at a distance of 1 cm to 60 cm from the arrangement for fixing the head of a person under test in a stationary position.

15. The device of claim 12, **characterized in that** the first screen is located at a distance of 1 cm to 60 cm from the arrangement for fixing the head of a person under test in a stationary position.

16. The device of anyone of claims 9 or 10, **characterized in that** it is provided with a processing unit connected to the means for creating an image on the first screen, to the means for creating an image on the second screen and to the means for recording movements of at least one eye pupil of said person under test.
